# EUROPÄISCHE PATENTSCHRIFT

(11) **EP 3 796 356 B1**
(45) Veröffentlichungstag und Bekanntmachung des Hinweises auf die Patenterteilung: **29.05.2024**
(21) Anmeldenummer: 20197773.3
(22) Anmeldetag: 23.09.2020
(51) Int. Cl.: H01H 9/18, H01H 13/16

(54) **FUSSSCHALTER FÜR MEDIZINISCHE GERÄTE**
FOOT SWITCH FOR MEDICAL DEVICES
INTERRUPTEUR À COMMANDE AU PIED POUR APPAREILS MÉDICAUX

(30) Priorität: 23.09.2019 DE 102019125490
(43) Veröffentlichungstag der Anmeldung: 24.03.2021
(73) Patentinhaber: Karl Storz SE & Co. KG, 78532 Tuttlingen (DE)
(72) Erfinder: Sabo, Alexander, 78532 Tuttlingen (DE); Zimmermann, Carina, 78532 Tuttlingen (DE)
(74) Vertreter: Weickert, Jonas

(56) Entgegenhaltungen:
- US-A- 5 635 777
- US-A1- 2007 227 868
- US-A1- 2010 198 200
- US-A1- 2013 168 212

## Beschreibung

Die vorliegende Erfindung ist auf einen Fußschalter zur Steuerung von einem oder mehreren medizinischen Geräten bezogen.

Zahlreiche medizinische Geräte weisen Funktionen auf, die durch medizinisches Personal ausgelöst, an- und ausgeschaltet, verändert, moduliert oder auf andere Weise gesteuert werden können. Dazu sind an medizinischen Geräten Benutzerschnittstellen vorgesehen oder die medizinischen Geräte mit Benutzerschnittstellen koppelbar. Für viele Anwendungen sind Fußschalter üblich, um medizinischem Personal eine Steuerung von einer oder mehreren Funktionen auch dann zu ermöglichen, wenn keine Hand für eine manuelle Einwirkung auf eine Benutzerschnittstelle frei ist.

Aus dem Stand der Technik ist bekannt, die Pedale medizinischer Fußschalter einzufärben, ihnen also feste Farben zuzuordnen, die die Funktion des Pedals anzeigen. So ist es üblich, mit Hochfrequenzchirurgie-Geräten verbundene Fußschalter mit blauen und gelben Pedalen zu versehen. Die Farben der Pedale lassen sich nicht ändern.

Aus der US7,557,317B2 ist ein medizinischer Fußschalter bekannt. Dieser kann mit einem elektrochirurgischen Gerät verbunden sein. Das Gehäuse des Fußschalters kann ein oder mehrere Lichtquellen aufweisen, die die am Gehäuse befestigten Pedale des Fußschalters beleuchten. Die Pedale weisen LEDs (Leuchtdioden) auf, die eine Funktion des betreffenden Pedals anzeigen. Die einzelnen LEDs können unterschiedlicher Farbe sein, einen Schriftzug von hinten beleuchten oder diesen Schriftzug bilden. Ebenfalls kann eine ausgewählte Funktion des Geräts auf einem Display am Fußschalter angezeigt werden.

Aus der US 2010/198200 A1 ist ein medizinischer Fußschalter bekannt, der über mehrere LED zur Anzeige eines Status eines verbundenen medizinischen Geräts oder des Fußschalters selbst verfügt. In verschiedenen Ausführungsformen kann das Gerät (z. B. ein chirurgischer Fußschalter, ein Handstück usw.) und eine chirurgische Konsole von einem Bediener während eines chirurgischen Eingriffs (z. B. eines augenchirurgischen Eingriffs) und/oder in einem Verfahren (z. B. zur Untersuchung eines Patienten, zur Kalibrierung des Geräts usw.) verwendet werden. Das Gerät kann beispielsweise über eine Schnittstelle mit der chirurgischen Konsole kommunizieren, um Informationen im Zusammenhang mit dem Verfahren zu erhalten (z. B. Informationen über das Gerät, die Konsole oder andere bei dem Verfahren verwendete Ausrüstung). Weiter können die Informationen im Gerät ermittelt/erzeugt werden (d. h. sie müssen nicht unbedingt von der chirurgischen Konsole empfangen werden). Auf der Grundlage der Informationen kann eine Konfiguration (z. B. am Gerät, an der Konsole usw.) für eine oder mehrere Indikatoren am Gerät festgelegt werden, um dem Bediener des Geräts zumindest einen Teil der Informationen zu liefern. Es kann ein Steuersignal erzeugt werden, um den mindestens einen Indikator entsprechend der festgelegten Konfiguration zum Leuchten zu bringen. Der mindestens eine Indikator auf dem Gerät kann also entsprechend dem übermittelten Steuersignal aufleuchten, um dem Bediener zumindest einen Teil der mit dem Verfahren (z. B. chirurgisches Verfahren, Untersuchungsverfahren, Kalibrierung usw.) verbundenen Informationen zu liefern.

Aus der US 5 635 777 A ist ein medizinischer Fußschalter bekannt, der mehrere Leuchten zur Anzeige von mehreren Funktionen eines verbundenen medizinischen Geräts verfügt, wobei jede Leuchte einer bestimmten Funktion zugeordnet ist.

Nachteilig an diesen aus dem Stand der Technik bekannten Fußschalter ist, dass nur eine sehr begrenzte Zahl von Funktionen durch die LEDs angezeigt und unterschieden werden kann. Andererseits ist das Vorsehen eines Displays zur Anzeige von Text auf dem Fußschalter sehr aufwändig und teuer.

Es ist daher Aufgabe der vorliegenden Erfindung einen verbesserten Fußschalter zu schaffen, dem eine große Zahl verschiedener Funktionen zugeordnet werden kann.

Diese Aufgabe wird durch ein erfindungsgemäßes medizinisches System mit einem Fußschalter und einem medizinischen Gerät gelöst, das in Anspruch 1 definiert ist.

Vorteilhafte Weiterbildungen der Erfindung ergeben sich aus den Unteransprüchen.

Der Fußschalter zur Verwendung mit einem medizinischen Gerät weist eine Schnittstelle zur kommunikativen Verbindung des Fußschalters mit einer Steuereinheit des Geräts auf. Über wenigstens ein Pedal des Fußschalters kann dieser von einem Anwender mit einem Fuß betätigt werden. Ein Betätigungssensor erfasst die Betätigung des Fußschalters, um so eine Funktion des medizinischen Geräts zu schalten. Der Betätigungssensor erzeugt hierfür ein entsprechendes, beispielsweise elektrisches, Signal, das über die Schnittstelle an die Steuereinheit des Geräts übertragen wird und dort das Schalten der Funktion des Geräts auslöst. Ferner weist der Fußschalter einen Prozessor auf, der dazu ausgebildet ist über die Schnittstelle Signale von der Steuereinheit zu empfangen, die dem wenigstens einen Pedal die zu schaltende Funktion zuordnen. Zudem ist an dem Fußschalter eine LED angeordnet, zur Anzeige der zugeordneten Funktion in einer bestimmten Farbe. Die LED ist hierbei eine mehrfarbige LED, die Licht in einer Vielzahl unterschiedlicher Farben erzeugen kann. Auf diese Weise werden die unterschiedlichen, dem Fußschalter jeweils zugeordneten Funktionen des Geräts in jeweils einer bestimmten Farbe durch die LED angezeigt.

Der Fußschalter kann über ein, zwei oder mehrere Pedale verfügen, die das über die Schnittstelle verbundene Gerät oder einzelne Funktionen dieses Geräts steuern. Üblich sind beispielsweise Fußschalter mit zwei Pedalen. Das Pedal ist beispielsweise als Wippe ausgebildet, mit einer Trittfläche, über die der Anwender mit dem Fuß das Pedal betätigen kann. Die Betätigung des Pedals, d.h. die Bewegung der Trittfläche, wird über einen oder mehrere Sensoren erfasst. Beispielsweise haben sich hier Hall-Sensoren bewährt, insbesondere solche, die eine Magnetfeldänderung präzise erfassen können. Die Sensoren können mehrfach vorgesehen sein, um Signale miteinander abzugleichen und zur Sicherheit des Patienten Fehlfunktionen zu vermeiden. Fußschalter können Druckpunkte aufweisen, so dass bei der Betätigung des Pedals ein Widerstand überwunden werden muss, der dem Anwender ein besseres Gefühl für die Betätigung des Pedals gibt und ein versehentliches Betätigen vermeidet. Der Fußschalter kann über ein Kabel mit Strom versorgt werden oder auch über eine eigene Stromquelle wie eine Batterie verfügen.

In der Medizin existiert eine Vielzahl von Geräten, die über Fußschalter gesteuert werden können, beispielsweise Pumpen für Flüssigkeiten, Insufflatoren zur Insufflation von Gas, Schlauchquetschventile, Kamerasysteme, Lichtquellen, Geräte zur Dokumentation von medizinischen Eingriffen, Geräte für die Hochfrequenzchirurgie, Steuergeräte für Laser, Motoren oder Lithotriptoren. Die zu steuernden Funktionen sind relevant für die Gesundheit des Patienten und potenziell gefährlich, so dass eine korrekte Bedienung der Geräte von großer Bedeutung ist.

Fußschalter und Gerät bzw. dessen Steuereinheit können zur Kommunikation und zum Austausch von Signalen über Kabel oder auch drahtlos über die Schnittstelle am Fußschalter verbunden werden. Im Fall einer drahtlosen Verbindung wird die Steuereinheit bei der Kommunikation mit dem Fußschalter sicherstellen, dass der richtige Fußschalter angeschlossen ist und nur dieser das Gerät schalten darf. Auch wird der Fachmann Mittel vorsehen, um die korrekte Verbindung zwischen Gerät und Fußschalter zu überwachen. Bricht beispielsweise die drahtlose Verbindung ab, so kann die Funktion des Geräts sicherheitshalber abgeschaltet oder gestoppt werden.

Erfindungsgemäß ist nun vorgesehen, dass die Steuereinheit des angeschlossenen Geräts dazu eingerichtet ist, dem Fußschalter für das wenigstens eine Pedal über die zur Kommunikation vorgesehene Schnittstelle eine Funktion des Geräts zuzuweisen, die über das Pedal gesteuert bzw. geschaltet werden soll. Die Signale werden hierfür über die Schnittstelle zum Prozessor des Fußschalters geleitet. Der Fußschalter ist also ein universell einsetzbarer Fußschalter, dem vorab keine feste Funktion zugewiesen ist, sondern dem je nach Anwendungsfall und Gerät vom angeschlossenen Gerät eine Funktion zugeordnet wird. Dies kann beim Anschluss des Fußschalters an das Gerät automatisch durch die Steuereinheit des Geräts erfolgen. Alternativ kann einem bereits angeschlossenen Fußschalter von einem Anwender über eine Bedieneinheit am Gerät oder der Steuereinheit eine oder mehrere Funktionen für die ein oder mehreren Pedale zugewiesen werden. Wiederum alternativ kann die Steuereinheit beim Einstellen eines bestimmten Betriebsmodus für das Gerät dem Fußschalter bzw. dessen Pedal eine dafür vorprogrammierte Funktion zuweisen. Allgemein kann die Steuereinheit Teil des medizinischen Geräts sein, sie kann jedoch auch eine separate Einheit sein, die beispielsweise auch noch weitere Geräte in einem medizinischen System steuert. Zur Kommunikation und zur Steuerung des Fußschalters selbst kann dieser einen Mikrocontroller aufweisen. Der Prozessor kann Teil des Mikrocontrollers sein, wie dies bei Mikrocontrollern üblicherweise vorgesehen ist. Das Gerät und die Steuereinheit können in bekannter Weise Prozessoren, Speicher, Software und Bedieneinheiten wie Schalter, Touchdisplays oder andere Eingabemittel aufweisen.

Die Betätigung des Pedals am Fußschalter durch den Anwender führt zu einem vom Betätigungssensor ausgelösten Bediensignal, das über die Schnittstelle an die Steuereinheit des Geräts übermittelt wird und dort die zuvor zugewiesene Funktion auslöst oder beendet. Mit dem Begriff "schalten" ist im vorliegenden Text sowohl das Auslösen als auch das Beenden einer Funktion oder das Wechseln zu einer anderen Funktion gemeint.

Da jedem Pedal des Fußschalters von der Steuereinheit eine Funktion individuell zugewiesen werden kann, ist vorgesehen, dass dem Anwender die zugewiesene Funktion durch eine farbig leuchtende LED (Light Emitting Diode, Leuchtdiode) am Fußschalter angezeigt wird. Es wird eine mehrfarbige LED verwendet, die in vielen unterschiedlichen Farben leuchten kann. So kann die mehrfarbige LED je nach Funktion des Geräts in einer bestimmten Farbe leuchten und dem Anwender die dem Pedal zugewiesene Funktion mitteilen. Es kann also jede Funktion durch eine eigene Farbe dargestellt werden, obwohl nur eine LED verwendet wird. Viele verschiedene Funktionen des Geräts oder mehrerer verbundener Geräte können dem Fußschalter bzw. den einzelnen Pedalen abwechselnd zugewiesen und jeweils durch eine eigene Farbe durch die mehrfarbige LED angezeigt werden. Dabei kann es sich um vorgegebene Farben handeln, die den gewünschten Funktionen üblicherweise zugeordnet werden oder durch eine Norm vorgeschrieben sind, wie Blau und Gelb für die Verwendung von Fußschaltern in der Hochfrequenzchirurgie für die Funktionen Koagulieren und Schneiden. Es kann sich jedoch auch um eine individuell vom Nutzer ausgewählte Farbe handeln, die dieser der speziellen gewünschten Funktion an der Steuereinheit manuell zuordnet. Bei mehreren Funktionen wird der Anwender verschiedene Farben auswählen. Alternativ sind geräteseitig bereits Farben für die unterschiedlichen Funktionen vorprogrammiert und werden dem Fußschalter mitgeteilt.

Die mehrfarbige LED kann aus mehreren, insbesondere wenigstens zwei Leuchtdioden bestehen oder es kann sich um eine einzige Leuchtdiode handeln. Die Leuchtdioden können in einem gemeinsamen LED-Gehäuse angeordnet sein. Die Leuchtdioden der mehrfarbigen LED können über eine gemeinsame Kathode oder eine gemeinsame Anode verbunden sein. Alternativ kann die mehrfarbige LED nur zwei verschiedene Dioden umfassen.

Die mehrfarbige LED kann insbesondere alle Farben eines RGB-Farbraums wiedergeben. Hierfür kann die mehrfarbige LED eine RGB-LED sein, also eine LED, die aus drei einzelnen Leuchtdioden besteht, die mit einstellbarer Intensität in den Farben Rot, Grün und Blau leuchten und zusammen alle Farben eines RGB-Farbraums erzeugen können. Die drei einzelnen Leuchtdioden werden dafür mit unterschiedlicher Intensität betrieben und kombiniert. Dem menschlichen Betrachter erscheint das Licht wie eine Farbe, die aus den drei Grundfarben gemischt wurde. Die mehrfarbige LED und insbesondere die RGB-LED hat den Vorteil, dass eine große Vielzahl von Funktionen unterschieden werden kann und diesen Funktionen individuelle Farben zugeordnet werden können, die dann nach der Zuordnung am Fußschalter angezeigt werden. Damit ist der Fußschalter für jede denkbare Anwendung geeignet und kann individuell verwendet werden.

Auch in einem komplexen medizinischen System mit vielen Geräten und Funktionen, die über den Fußschalter gesteuert werden sollen, lassen sich die Pedale so eindeutig einer Funktion zuordnen. So kann der Fußschalter wenigstens zwei Pedale und zwei mehrfarbige LEDs aufweisen, wobei den Pedalen von der Steuereinheit unterschiedliche Funktionen zugewiesen werden. Diese Funktionen werden dann über die jeweilige mehrfarbige LED farbig angezeigt. Üblicherweise wird jedem Pedal eine eigene mehrfarbige LED zugeordnet sein.

Der Anwender sieht auf einen Blick, welche Funktion einem Pedal zugeordnet ist und eine Fehlbedienung wird vermieden. Auch kann der Fußschalter insbesondere bei einer drahtlosen Verbindung im Wechsel mit unterschiedlichen Geräten verbunden werden, die den Pedalen die jeweils passende Funktion zuordnen und diese am Fußschalter mit der entsprechenden Farbe anzeigen lassen. Dies erhöht die Flexibilität des Systems und der Anwendung. Auch benötigt man nicht mehr einen Fußschalter für jede Funktion.

Der Fußschalter kann ein Gehäuse aufweisen, in oder an dem das eine oder die mehreren Pedale angeordnet sind. In diesem Fall kann sich die mehrfarbige LED im Gehäuse befinden und mit einem transparenten Sichtfenster abgedeckt sein, durch welches ein Anwender die von der mehrfarbigen LED erzeugte Lichtfarbe sehen kann. Das Sichtfenster kann in der Oberfläche des Gehäuses angeordnet sein und beispielsweise bündig mit diesem abschließen. Beispielsweise kann sich die mehrfarbige LED in einem Bereich des Gehäuses oberhalb des Pedals befinden. Das Sichtfenster ist dann in der Oberseite des Gehäuses angeordnet. Auch andere Abdeckungen der LEDs sind denkbar. Ebenso können sich diese statt im Gehäuse in der Trittfläche des Pedals befinden. Dies hat jedoch den Nachteil, dass das farbige Licht bei Betätigung durch den Fuß möglicherweise nicht mehr zu sehen ist.

Das Gehäuse des Fußschalters kann weiterhin einen zusätzlichen Betätigungsschalter aufweisen, über den sich weitere Funktionen schalten lassen. Beispielsweise könnte damit der Fußschalter aktiviert und deaktiviert oder die drahtlose Verbindung aktiviert oder deaktiviert werden. Der Schalter kann ebenfalls durch eine insbesondere mehrfarbige LED beleuchtet werden, die den Schalter selbst erleuchtet, oder der Schalter kann einen Rand aufweisen, beispielsweise einen Ring, der den Schalter umgibt, der von einer LED erleuchtet wird. So lassen sich der Aktivierungszustand des Fußschalters oder der drahtlosen Verbindung oder auch Fehlermeldungen durch verschiedene Farben oder Signale wie Blinken darstellen, um den Anwender zu informieren.

Zur Kommunikation zwischen Fußschalter und Gerät bzw. dessen Steuereinheit können beide Teile über ein medizinisches Datenübertragungssystem, insbesondere ein BUSSystem verbunden sein. Dieses kann wiederum Teil eines größeren medizinischen Systems mit vielen Komponenten sein, wie dies in einem Operationssaal der Fall ist. Die Steuereinheit kann dann auch Teil der Steuerung des Operationssaales sein. Es kann vorgesehen sein, dass der Fußschalter bei Aktivierung oder Verbindung mit dem System automatisch erkannt wird und ihm bereits vorausgewählte Funktionen von der Steuereinheit zugewiesen werden, die dann durch die mehrfarbigen LEDs angezeigt werden.

Alternativ können ein Gerät mit Steuereinheit und ein Fußschalter über andere kabelgebundene oder drahtlose Signalwege miteinander verbunden sein und die Steuereinheit kann über einen BUS oder eine andere Datenverbindung mit einem medizinischen System verbunden sein.

Die Erfindung umfasst daher auch ein medizinisches System mit wenigstens zwei Geräten und wenigstens einem vorstehend beschriebenen Fußschalter, wobei die Geräte und der wenigstens eine Fußschalter über ein medizinisches Datenübertragungssystem verbunden sind und über dieses kommunizieren.

Weitere Aspekte der Erfindung ergeben sich aus der nachfolgenden Beschreibung der bevorzugten Ausführungsbeispiele und den beigefügten Zeichnungen. Es zeigen:
- Figur 1: einen Fußschalter in Verbindung mit einem Gerät
- Figur 2: einen Fußschalter in drahtloser Verbindung mit einem Gerät
- Figur 3: eine schematische Darstellung des Fußschalters
- Figur 4: ein medizinisches System mit Geräten und Fußschaltern

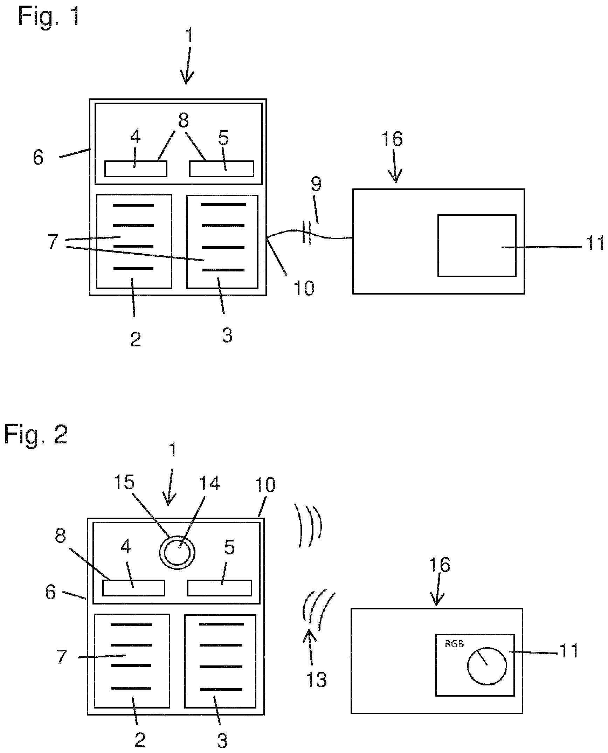

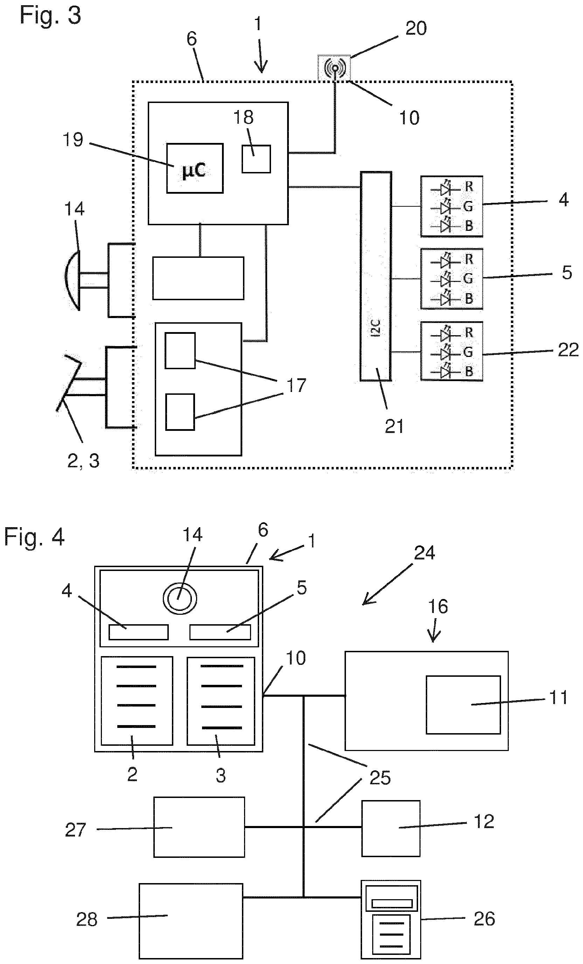

In Figur 1 ist schematisch ein erfindungsgemäßer Fußschalter 1 mit zu bedienendem Gerät 16 dargestellt. Der Fußschalter 1 umfasst ein Gehäuse 6, in dem ein erstes Pedal 2 und ein zweites Pedal 3 jeweils mit einer Trittfläche 7 angeordnet sind. Die Trittfläche hat eine geriffelte Oberfläche für besseren Halt. In einem Bereich des Gehäuses 6 oberhalb der Pedale 2 und 3 befinden sich Sichtfenster 8, hinter denen sich eine erste mehrfarbige LED 4 und eine zweite mehrfarbige LED 5 befinden. Das Licht der LEDs 4 und 5 dringt durch das jeweilige Sichtfenster 8 nach außen und ist für einen Anwender erkennbar. Die Pedale 2, 3 werden in bekannter Weise von einem Anwender mit dem Fuß bedient, um das Gerät 16 zu steuern. Zu diesem Zweck weisen die Pedale 2, 3 jeweils wenigstens einen Betätigungssensor 17 auf, der in Figur 1 nicht dargestellt ist. Der Sensor 17 erfasst die Betätigung des jeweiligen Pedals 2, 3 durch den Anwender und leitet das Signal an einen Mikrocontroller 19 im Fußschalter 1 weiter. Dort wird das Signal von einem nicht gesondert dargestellten Prozessor verarbeitet. Der Aufbau der Schaltung der verschiedenen Komponenten innerhalb des Fußschalters 1 ist in Figur 3 schematisch dargestellt. Der Betätigungssensor 17 ist beispielsweise ein linearer Hall-Sensor der Magnetfeldänderungen misst. Dieser kann beispielsweise die Bewegung der Trittfläche 7 oder die Bewegung einer Drehachse des Pedals 2, 3 präzise erfassen.

Über eine Schnittstelle 10 und ein Kabel 9 kommuniziert der Fußschalter 1 mit einem verbundenen Gerät 16 und dessen Steuereinheit 12 (hier nicht dargestellt). Dieses Gerät 16 kann beispielsweise eine medizinische Saugpumpe sein, die der Entfernung von unerwünschten Flüssigkeiten oder Gasen aus einer Körperhöhle dient. Der Anwender betätigt den Fußschalter 1 und aktiviert oder deaktiviert die Saugfunktion der Pumpe 16, beispielsweise wenn die Sicht in die Körperhöhle durch Flüssigkeit oder Gas beeinträchtigt ist. Die Steuereinheit 17 befindet sich in diesem Beispiel innerhalb des Geräts 16 und ist Teil dieses Geräts. Auf einem Touchdisplay 11 wird die eingestellte Stärke der Saugfunktion der Pumpe angezeigt.

Welche Funktion die Pedale 2, 3 des Fußschalters 1 ausführen sollen, wird dem Fußschalter 1 von der Steuereinheit 12 über die Schnittstelle 10 mitgeteilt. Der Fußschalter 1 ist hierfür eingerichtet, von der Steuereinheit 12 Signale oder Befehle über die Schnittstelle 10 zu empfangen. Diese werden von einem Mikrocontroller 19 verarbeitet (s. Figur 3).

Im vorliegenden Beispiel sei die Aktivierung der Pumpe dem Pedal 2 und die Deaktivierung dem Pedal 3 zugeordnet. Die oberhalb des Pedals 2 angebrachte mehrfarbige LED 4 leuchtet beispielsweise in einem Orangeton, die LED 5 oberhalb des Pedals 3 beispielsweise in einem Lilaton. Die hier verwendeten RGB-LEDs können in den verschiedenen Farben des RGB-Farbraums leuchten. Diese Farben sind den Funktionen zugeordnet und teilen dem Anwender eindeutig mit, welche Funktion durch die Bedienung der Pedale 2 und 3 im Gerät 16 ausgelöst wird. Auf diese Weise kann eine Fehlbedienung des Gerätes 16 vermieden werden.

Figur 2 zeigt ein weiteres Ausführungsbeispiel eines Fußschalters 1. Die Komponenten sind wie zuvor beschrieben, jedoch sind der Fußschalter 1 und die Steuereinheit 12 des Geräts 16 nun nicht über ein Kabel, sondern über eine drahtlose Signalverbindung 13 in Kontakt. Zu diesem Zweck weisen Fußschalter 1 und Gerät 16 hier nicht dargestellte Sender und Empfänger auf. Der Fußschalter 1 umfasst nun zusätzlich einen Betätigungsschalter 14, der auf dem Gehäuse 6 des Fußschalters 1 angeordnet ist. Der Schalter 14 kann ebenfalls mit einem Fuß bedient werden und ist beispielsweise ein Druckschalter. Betätigen des Schalters 14 aktiviert den Fußschalter 1, so dass dieser zur Verfügung steht. Der Betätigungsschalter 14 ist von einem Ring 15 umgeben, hinter dem wiederum eine mehrfarbige LED 22 angebracht ist. Der Ring 15 ist transparent und kann dank der LED 22 in verschiedenen Farben leuchten. Durch unterschiedliche Farben wird dem Anwender der Status des Fußschalters 1 mitgeteilt, beispielsweise ob dieser aktuell über die drahtlose Verbindung 13 mit dem Gerät 16 und dessen Steuereinheit 12 in Kontakt steht. Der Fußschalter 1 kann optional in der Lage sein, nach längerem Nichtgebrauch in einen Ruhemodus zu schalten. Dieser Ruhemodus kann ebenfalls über eine festgelegte Farbe im Ring 15 angezeigt werden.

Wenn der Anwender dem Fußschalter 1 und dessen Pedalen 2 und 3 nun andere Farben für die Funktionen "Aktivieren" und "Deaktivieren" zuordnen möchte, beispielsweise um sie deutlicher von den Farben eines anderen Fußschalters zu unterscheiden oder weil Farben einem Anwender besser erkennbar erscheinen, kann der Anwender die den Funktionen zugeordneten Farben über das Touchdisplay 11 am Gerät 16 ändern. Da es sich bei den mehrfarbigen LEDs 4 und 5 um RGB-LEDs handelt, stehen dem Anwender die Farben eines RGB-Farbraums zur Verfügung und können nach Bedarf gewählt werden. Nach der Auswahl teilt die Steuereinheit 12 dem Fußschalter 1 über die Wireless-Verbindung 13 und die Schnittstelle 10 mit, welche Farben den Funktionen "Aktivieren" und "Deaktivieren" zugeordnet und bei den Pedalen 2 und 3 durch die LEDs 4 und 5 angezeigt werden sollen. Der Mikrocontroller 19 im Fußschalter 1 regelt die LEDs 4 und 5 entsprechend (s. auch Figur 3).

Die Schaltung der Komponenten innerhalb des Fußschalters 1 ist schematisch in der Figur 3 dargestellt. Das Gehäuse 6 des Fußschalters 1 ist hier durch eine gestrichelte Linie angedeutet. Schematisch sind an diesem Gehäuse auch die Pedale 2, 3 und der Betätigungsschalter 14 dargestellt. Den Pedalen 2, 3 ist jeweils ein Betätigungssensor 17 zugeordnet, der die Betätigung der Pedale an einen Mikrocontroller 19 meldet. Ein Lagesensor 18 erfasst, wenn sich der Fußschalter 1 nicht mehr in einer waagerechten Position befindet, so wie sie für eine zuverlässige Bedienung des Fußschalters 1 notwendig ist, und veranlasst den Mikrocontroller 19 gegebenenfalls den Fußschalter sicherheitshalber zu deaktivieren. Mit dem Mikrocontroller 19 steht auch die Schnittstelle 10 mit der Antenne 20 in Verbindung, die die drahtlose Kommunikation mit dem Gerät 16 ermöglicht. In Abhängigkeit der Anweisungen der Steuereinheit 12 des Geräts 16 steuert der Mikrocontroller 19 über den LED-BUS 21 (I2C) die RGB-LEDs 4 und 5, sowie die dritte RGB-LED 22, die dem Ring 15 des Betätigungsschalters 14 zugeordnet ist. Durch geeignetes Steuern der Intensitäten der einzelnen R-, G- und B-Dioden lassen sich die verschiedenen Farben des RGB-Farbraums erzeugen. Die drei Dioden sind jeweils in einem gemeinsamen LED-Gehäuse angeordnet und über einen gemeinsamen Eingang elektrisch verbunden.

Tritt bei der drahtlosen Verbindung 13 beispielsweise eine Störung auf, so dass eine sichere Kommunikation zwischen Fußschalter 1 und Gerät 16 nicht mehr gewährleistet ist, so steuert der Mikrocontroller 19 die LED 22 derart, dass sie in einer definierten Warnfarbe leuchtet und den Anwender über das Problem informiert. Es versteht sich, dass der Betätigungsschalter 14 und die ihm zugeordnete dritte mehrfarbige LED 22 optional sind und in anderen Ausführungsformen nicht vorgesehen sein müssen, wie dies beispielsweise zu Figur 1 ausgeführt wurde.

Die besonderen Vorteile des Fußschalters 1 zeigen sich auch in Figur 4. Hier ist ein medizinisches System 24 dargestellt, mit einem Fußschalter 1 und einem Gerät 16 wie zuvor beschrieben, sowie mit einem zweiten Gerät 27, einem dritten Gerät 28, einer Steuereinheit 12 und einem zweiten Fußschalter 26. Dieser Fußschalter 26 ist erfindungsgemäß wie der Fußschalter 1 aus dem Ausführungsbeispiel der Figur 1 gestaltet, jedoch weist er nur ein Pedal und eine mehrfarbige LED für dieses Pedal auf. Die anderen Komponenten wie Gehäuse, Pedal, Trittfläche und innerer Aufbau sind analog dem Fußschalter 26 angeordnet. Die Geräte 16, 27 und 28 und die Fußschalter 1 und 26 sind über einen medizinischen Daten-BUS 25 untereinander verbunden. Ebenfalls Teil dieses Systems 24 und mit dem BUS 25 verbunden ist die Steuereinheit 12, die im vorliegenden Beispiel nicht innerhalb des Geräts 16 angeordnet ist, sondern eine separate Einheit mit eigenem Gehäuse bildet. Die Steuereinheit 12 ist hier eine Operationssaal-Steuerung, die über den BUS 25 auf alle Geräte und Fußschalter im System zugreift und die Steuerung der Geräte über die Fußschalter steuert. Insbesondere weist die Steuereinheit 12 den Fußschaltern 1 und 26 über deren Schnittstellen und den BUS 25 die verschiedenen Gerätefunktionen der Geräte 16, 27 und 28 zu, die mit den verschiedenen Pedalen gesteuert werden sollen. Außerdem weist die Steuereinheit 12 den benötigten Funktionen die Farben zu, die an den Fußschaltern 1 und 26 den Pedalen zugeordnet angezeigt werden sollen. Die Steuereinheit 12 kann hier automatisch oder manuell über den Anwender den Funktionen individuelle Farben zuordnen, die die Funktionen und Pedale voneinander unterscheidbar machen und eindeutig anzeigen, welches Pedal welche Funktion der Geräte 16, 27 und 28 steuert. Nach Bedarf können die Farben und Funktionen den Fußschaltern 1 und 26 und deren Pedalen neu zugeordnet werden. Alternativ kann die Steuereinheit 12 die Zuordnung sofort vornehmen, wenn die Fußschalter 1 und 26 mit dem BUS 25 und dem System 24 verbunden werden. Das System 24 lässt sich beliebig erweitern und den Fußschaltern 1 und 26 wie erfindungsgemäß vorgesehen eine große Zahl verschiedener Funktionen zuordnen, die durch unterschiedliche Farben gekennzeichnet, an den Fußschaltern angezeigt und voneinander unterschieden werden können.

### Bezugszeichenliste:

- 1: Fußschalter
- 2: erstes Pedal
- 3: zweites Pedal
- 4: erste mehrfarbige LED
- 5: zweite mehrfarbige LED
- 6: Gehäuse
- 7: Trittfläche
- 8: Sichtfenster
- 9: Kabel
- 10: Schnittstelle
- 11: Touchdisplay
- 12: Steuereinheit
- 13: Wireless-Verbindung
- 14: Betätigungsschalter
- 15: Ring
- 16: Gerät
- 17: Betätigungssensor
- 18: Lagesensor
- 19: Mikrocontroller
- 20: Antenne
- 21: LED-BUS
- 22: dritte mehrfarbige LED
- 24: medizinisches System
- 25: BUS
- 26: zweiter Fußschalter
- 27: zweites Gerät
- 28: drittes Gerät

## Patentansprüche

1. Medizinisches System (24) mit einem medizinischen Gerät (16, 27, 28) und mit einem Fußschalter (1, 26) zur Verwendung mit dem medizinischen Gerät (16, 27, 28),
der Fußschalter (1, 26) umfassend:
eine Schnittstelle (10) zur kommunikativen Verbindung des Fußschalters (1, 26) mit einer Steuereinheit (12) des Geräts (16, 27, 28),
wenigstens ein Pedal (2, 3) zur Betätigung des Fußschalters (1, 26) mit einem Fuß,
einen Betätigungssensor (17), der dazu ausgebildet ist eine Betätigung des Fußschalters (1, 26) zu erfassen,
wobei der Betätigungssensor (17) ein Signal erzeugt, das über die Schnittstelle (10) an die Steuereinheit (12) des Geräts (16, 27, 28) übertragen wird, zum Schalten einer Funktion des medizinischen Geräts (16, 27, 28),
einen Prozessor, der dazu ausgebildet ist über die Schnittstelle (10) Signale von der Steuereinheit (12) zu empfangen, die dem wenigstens einen Pedal (2, 3) die zu schaltende Funktion zuordnen, und
wenigstens eine LED, zur Anzeige der zugeordneten Funktion in einer bestimmten Farbe,
wobei die LED eine mehrfarbige LED (4, 5) ist, die Licht in einer Vielzahl unterschiedlicher Farben erzeugen kann, zur Anzeige unterschiedlicher Funktionen des Geräts (16, 27, 28).

2. Medizinisches System (24) nach Anspruch 1,
wobei die mehrfarbige LED (4, 5) wenigstens zwei Leuchtdioden umfasst, die eine gemeinsame Anode oder eine gemeinsame Kathode aufweisen.

3. Medizinisches System (24) nach einem der vorherigen Ansprüche,
wobei die mehrfarbige LED (4, 5) wenigstens zwei Leuchtdioden umfasst, die in einem gemeinsamen LED-Gehäuse angeordnet sind.

4. Medizinisches System (24) nach einem der vorherigen Ansprüche, wobei die mehrfarbige LED (4, 5) alle Farben eines RGB-Farbraums wiedergeben kann.

5. Medizinisches System (24) nach einem der vorherigen Ansprüche,
wobei der Fußschalter (1, 26) ein Gehäuse (6) aufweist, an dem das wenigstens eine Pedal (2, 3) und die wenigstens eine mehrfarbige LED (4, 5) angeordnet sind.

6. Medizinisches System (24) nach einem der vorherigen Ansprüche,
wobei der Fußschalter (1, 26) wenigstens zwei Pedale (2, 3) und wenigstens zwei mehrfarbige LEDs (4, 5) aufweist und den Pedalen (2, 3) von der Steuereinheit (12) des Geräts (16, 27, 28) unterschiedliche Funktionen zugeordnet werden können und die zugeordneten Funktionen jeweils durch eine der mehrfarbigen LEDs (4, 5) angezeigt werden.

7. Medizinisches System (24) nach einem der vorherigen Ansprüche,
wobei die mehrfarbige LED (4, 5) jeweils durch ein transparentes Sichtfenster (8) abgedeckt ist, durch welches ein Anwender die von der mehrfarbigen LED (4, 5) erzeugte Lichtfarbe sehen kann.

8. Medizinisches System (24) nach Anspruch 7,
wobei das Sichtfenster (8) in einer Fläche des Gehäuses (6) angeordnet ist.

9. Medizinisches System (24) nach einem der Ansprüche 5 bis 8,
wobei das Gehäuse (6) zusätzlich einen Betätigungsschalter (14) aufweist, der durch eine weitere Leuchtdiode (22) beleuchtet wird oder von einem von einer weiteren Leuchtdiode (22) beleuchteten Rand (15) umgeben ist.

10. Medizinisches System (24) nach einem der vorherigen Ansprüche,
wobei der Fußschalter (1, 26) und das Gerät (16, 27, 28) über ein medizinisches Datenübertragungssystem (25) verbunden sind und über dieses kommunizieren.

11. Medizinisches System (24) nach einem der vorherigen Ansprüche mit wenigstens einem weiteren Gerät (16; 27, 28),
wobei die Geräte (16, 27, 28) und der Fußschalter (1, 26) über ein medizinisches Datenübertragungssystem (25) verbunden sind und über dieses kommunizieren.

## Claims

1. Medical system (24) with a medical device (16, 27, 28) and with a foot switch (1, 26) for use with the medical device (16, 27, 28),
the foot switch (1, 26) comprising:
an interface (10) for communicative connection of the foot switch (1, 26) with a control unit (12) of the device (16, 27, 28),
at least one pedal (2, 3) for actuating the foot switch (1, 26) with a foot,
an actuation sensor (17), which is configured to detect an actuation of the foot switch (1, 26),
wherein the actuation sensor (17) generates a signal that is transmitted via the interface (10) to the control unit (12) of the device (16, 27, 28) for switching a function of the medical device (16, 27, 28),
a processor which is configured to receive signals from the control unit (12), which assigns the function that is to be switched to the at least one pedal (2, 3) via the interface (10), and
at least one LED to display the assigned function in a certain color,
wherein the LED is a multicolor LED (4, 5), which can generate light in a multiplicity of different colors to display different functions of the device (16, 27, 28).

2. Medical system (24) according to claim 1,
wherein the multicolor LED (4, 5) comprises at least two light-emitting diodes which have a common anode or a common cathode.

3. Medical system (24) according to any one of the preceding claims,
wherein the multicolor LED (4, 5) comprises at least two light-emitting diodes which are arranged in a common LED housing.

4. Medical system (24) according to any one of the preceding claims, wherein the multicolor LED (4, 5) can reproduce all colors of an RGB color space.

5. Medical system (24) according to any one of the preceding claims,
wherein the foot switch (1, 26) has a housing (6) on which the at least one pedal (2, 3) and the at least one multicolor LED (4, 5) are arranged.

6. Medical system (24) according to any one of the preceding claims,
wherein the foot switch (1, 26) has at least two pedals (2, 3) and at least two multicolor LEDs (4, 5) and different functions can be assigned to the pedals (2, 3) by the control unit (12) of the device (16, 27, 28) and the assigned functions are each displayed by one of the multicolor LEDs (4, 5).

7. Medical system (24) according to any one of the preceding claims,
wherein the multicolor LED (4, 5) is respectively covered by a transparent viewing window (8) through which a user can see the light color generated by the multicolor LED (4, 5).

8. Medical system (24) according to claim 7,
wherein the viewing window (8) is arranged in a plane of the housing (6).

9. Medical system (24) according to any one of claims 5 to 8,
wherein the housing (6) additionally has an actuation switch (14), which is illuminated by a further light-emitting diode (22) or which is surrounded by an edge (15) illuminated by a further light-emitting diode (22).

10. Medical system (24) according to any one of the preceding claims,
wherein the foot switch (1, 26) and the device (16, 27, 28) are linked via a medical data transmission system (25) and communicate via the latter.

11. Medical system (24) according to any one of the preceding claims with at least one other device (16; 27, 28),
wherein the devices (16, 27, 28) and the foot switch (1, 26) are linked via a medical data transmission system (25) and communicate via the latter.

## Revendications

1. Système médical (24) comprenant un appareil médical (16, 27, 28) et un interrupteur à commande au pied (1, 26) destiné à être utilisé avec l'appareil médical (16, 27, 28),
l'interrupteur à commande au pied (1, 26) comprenant :
une interface (10) pour la liaison de communication de l'interrupteur à commande au pied (1, 26) avec une unité de commande (12) de l'appareil (16, 27, 28),
au moins une pédale (2, 3) pour actionner l'interrupteur à commande au pied (1, 26) avec un pied,
un capteur d'actionnement (17) qui est conçu pour détecter un actionnement de l'interrupteur à commande au pied (1, 26),
dans lequel le capteur d'actionnement (17) génère un signal qui est transmis à l'unité de commande (12) de l'appareil (16, 27, 28) par l'intermédiaire de l'interface (10), pour commuter une fonction de l'appareil médical (16, 27, 28),
un processeur qui est conçu pour recevoir de l'unité de commande (12), par l'intermédiaire de l'interface (10), des signaux qui attribuent la fonction à commuter à ladite au moins une pédale (2, 3), et
au moins une DEL, pour indiquer la fonction attribuée dans une couleur déterminée,
la DEL étant une DEL multicolore (4, 5) qui peut générer de la lumière dans une pluralité de couleurs différentes, pour indiquer différentes fonctions de l'appareil (16, 27, 28).

2. Système médical (24) selon la revendication 1,
dans lequel la DEL multicolore (4, 5) comprend au moins deux diodes électroluminescentes qui ont une anode commune ou une cathode commune.

3. Système médical (24) selon l'une quelconque des revendications précédentes,
dans lequel la DEL multicolore (4, 5) comprend au moins deux diodes électroluminescentes qui sont disposées dans un boîtier de DEL commun.

4. Système médical (24) selon l'une quelconque des revendications précédentes, dans lequel la DEL multicolore (4, 5) peut reproduire toutes les couleurs d'un espace chromatique RVB.

5. Système médical (24) selon l'une quelconque des revendications précédentes,
dans lequel l'interrupteur à commande au pied (1, 26) présente un boîtier (6) sur lequel sont disposées ladite au moins une pédale (2, 3) et ladite au moins une DEL multicolore (4, 5).

6. Système médical (24) selon l'une quelconque des revendications précédentes,
dans lequel l'interrupteur à commande au pied (1, 26) présente au moins deux pédales (2, 3) et au moins deux DEL multicolores (4, 5) et différentes fonctions peuvent être attribuées aux pédales (2, 3) par l'unité de commande (12) de l'appareil (16, 27, 28) et les fonctions attribuées sont respectivement indiquées par une des DEL multicolores (4, 5).

7. Système médical (24) selon l'une quelconque des revendications précédentes,
dans lequel chaque DEL multicolore (4, 5) est recouverte par une fenêtre d'observation transparente (8) à travers laquelle un utilisateur peut voir la couleur de lumière générée par la DEL multicolore (4, 5).

8. Système médical (24) selon la revendication 7,
dans lequel la fenêtre d'observation (8) est disposée dans une surface du boîtier (6).

9. Système médical (24) selon l'une quelconque des revendications 5 à 8,
dans lequel le boîtier (6) présente en outre un interrupteur d'actionnement (14) qui est éclairé par une autre diode électroluminescente (22) ou qui est entouré par un bord (15) éclairé par une autre diode électroluminescente (22).

10. Système médical (24) selon l'une quelconque des revendications précédentes,
dans lequel l'interrupteur à commande au pied (1, 26) et l'appareil (16, 27, 28) sont reliés et communiquent par l'intermédiaire d'un système de transmission de données médicales (25).

11. Système médical (24) selon l'une quelconque des revendications précédentes, comprenant au moins un autre appareil (16 ; 27, 28),
dans lequel les appareils (16, 27, 28) et l'interrupteur à commande au pied (1, 26) sont reliés et communiquent par l'intermédiaire d'un système de transmission de données médicales (25).
